(19) Europäisches Patentamt
European Patent Office
Office européen des brevets

(11) EP 1 369 807 A1

(12) EUROPEAN PATENT APPLICATION

(43) Date of publication:
10.12.2003 Bulletin 2003/50

(51) Int Cl.$^7$: G06F 19/00

(21) Application number: 02291407.1

(22) Date of filing: 06.06.2002

(84) Designated Contracting States:
AT BE CH CY DE DK ES FI FR GB GR IE IT LI LU MC NL PT SE TR
Designated Extension States:
AL LT LV MK RO SI

(71) Applicant: CENTRE NATIONAL DE LA RECHERCHE SCIENTIFIQUE (CNRS)
75794 Paris Cedex 16 (FR)

(72) Inventors:
- Geourjon, Chrsitophe
  69007 Lyon (FR)
- Jambon, Martin
  69007 Lyon (FR)
- Deleage, Gilbert
  69008 Lyon (FR)

(74) Representative: Breesé, Pierre
BREESE - MAJEROWICZ,
3, avenue de l'Opéra
75001 Paris (FR)

# (54) Process for identifying similar 3d substructures onto 3d atomic structures

(57) The present invention pertains to the field of structural biology and relates to a process to compare various three-dimensional (3D) structures and to identify functional similarities among them. The process of comparison of 3D atomic structures of the invention is based on the comparisons of defined chemical groups onto the 3D atomic structures and allows the detection of local similarities even when neither the fold nor sequence for example aminoacid sequences for polypeptides sequences or nucleotide sequences for nucleic acid sequences are conserved. This process requires the attribution of selected physico-chemical parameters to each atom of a 3D atomic structure, then the representation of each 3D atomic structure by a graph of chemical groups.

EP 1 369 807 A1

## Description

The present invention pertains to the field of structural biology and relates to a process to compare various three-dimensional structures and to identify functional similarities among them. In particular this process applies to macromolecules, as for example proteins.

Understanding and predicting the function of proteins using bioinformatical tools traditionally uses three levels of knowledge: aminoacid sequence, backbone fold and local arrangement of atoms. Several tools dealing with sequence or main chain structure are publicly available through the World Wide Web and routinely used by molecular biologists: tools such as Blast [1] and Fasta [2] provide efficient ways to extract similar sequences from databases containing millions of sequences. There also exist tools that help to correlate sequence and function using sequential patterns: the Prosite database [3] consists of human-designed functional signatures that may be searched against a protein sequence.

Profile analysis [4] is a technique based on multiple sequence alignments of homologous sequences and may be used to test a sequence for its membership of a family. Pattinprot [5] allows to search a database for any given pattern, that may have been inferred from multiple sequence alignments such as those obtained with ClustalW [6] from a set of homologous protein sequences. When a 3D structure of a given protein is available, it is possible to use tools such as the Dali/FSSP server [7, 8] that mainly use the main chain to find similarities and classify proteins. But these process reach their limits in many cases: a significant similarity in the sequence or in the fold of two proteins is neither necessary nor sufficient to prove that they share a common biological function.

Inferring biological function from 3D structures of proteins is still a hard problem, given that it strongly depends upon the biological context surrounding every protein molecule in vivo. However, analyzing precisely data provided by crystallographic or NMR experimental studies may show local structural similarities across various proteins, that could be correlated to an already known biological function. Though a lot of efforts have been spent along the past years on the development of surface matching algorithms, very few methods combine chemical information together with geometry in an efficient manner, and none of them use custom chemical groups as the elementary bricks responsible for biochemical activity.

Methodologies based on computer vision heuristics have been developed in the 90's [9]. These processes are purely geometrical and use discretized representations of molecular surface. Variants and improvements of the original technique select sparse critical points among all points representing the molecular surface [10] and introduce a small number of hinges allowing flexibility in the docking or matching process [11]. Other tools use the surface representation of the proteins to perform comparisons by other means [12-14]. Chemical environment has been taken successfully into account in predictive studies concerning special cases: metal-binding sites [15, 16], sugar-binding sites [17]. Thus the challenge was to provide a generic tool that returns satisfying results for a large number of protein functions without manual nor statistical tuning.

Several methods implementing comparison of the protein sequences or folding has been already developed, nevertheless most of them are only able to reflect similarities found at the surface of the 3D objects.

The applicant has now developed a new process of identification of similarities between the 3D atomic structures, even if those similarities are not exposed over the surface of said 3D atomic structures.

The process developed by the applicant is not based as most of previous ones, neither in comparison of linear sequences nor in comparison of folding structures.

The process of comparison of 3D atomic structures of the invention is based on the comparisons of defined chemical groups onto the 3D atomic structures and allows the detection of local similarities even when neither the fold nor sequence for example aminoacid sequences for polypeptides sequences or nucleotide sequences for nucleic acid sequences are conserved. This process requires the attribution of selected physico-chemical parameters to each atom of a 3D atomic structure, then the representation of each 3D atomic structure by a graph of chemical groups, i.e. if said chemical groups are selected by forming triplets, then the graph is represented by triangles.

Then, the starting point of the representation of 3D atomic structures is the definition of chemical groups within the structure. Some atoms may not belong to any of the chemical groups, whereas some others may be part of several groups as illustrated by table 1.

Table 1 illustrates the definition of some chemical groups that may be used in the process of the invention, though other definitions could be specified by the user.

The chemical group description groups done in this table 1 show an example of correspondence between chemical groups and aminoacids. Column 3 shows amino acids that contain at least one of the given chemical group from column 1. Column 4 indicates the geometric construction that is associated to the given chemical group, as defined in Figure 3.

Table 1

| Chemical group description | Symbol | Amino acids | Geometry |
|---|---|---|---|
| Carboxylic acid | acyl | Asp, Glu | S4 |
| Primary amide | amide | Asn, Gln | S5 |
| Aromatic ring | aromatic | His, Phe, Trp, Tyr | S2 |
| Free $\delta^+$ hydrogen | delta_minus | all | S3 |
| Free $\delta^-$ atom | delta_plus | all except Pro | S3 |
| Glycine | glycine | Gly | S3 |
| Guanidinium group | guanidinium | Arg | S2 |
| Imidazole group | histidine | His | S5 |
| Hydroxyl group | hydroxyl | Ser, Thr, Tyr | S3 |
| Proline | proline | Pro | S1 |
| Methionine sulphur | thioether | Met | S1 |
| Cysteine thiol | thiol | Cys | S3 |

Before any comparison, the 3D atomic structures have to be preformatted. This operation takes usually longer than the comparison itself. Thus, the preformatted data may be stored into a database in order to be reused later.

**1- Chemical groups construction**

Chemical groups are defined as sets of atoms that share strong geometric constraints and in a way that focuses on their potential interaction with target molecules.
Hydrogen bond donors, hydrogen bond acceptors, aromatic rings are examples of potential interactors with target molecules and thus may constitute chemical groups within the meaning of the present invention.
For the purpose of the present invention, chemical groups are 3D atomic substructures having a common set of physico-chemical parameters including:

I. Spatial position of their center and
II. Group-specific information

Additional geometric information is associated to each chemical group. The form of this information is specific to each kind of chemical group, since it is only required for the comparison and the scoring of chemical groups of the same kind.

The following geometric objects could be used to represent this group-specific information (figure 3):

S1: empty information. This could be used to represent isotropic objects such as a charge.
S2: non oriented symmetry axis . This could be used to reflect symmetric bipolar objects such as aromatic rings.
S3: simple polarisation. This represents an orientation in a single direction This representation may be useful to represent hydrogen bond donors and acceptors.
S4: semi-symmetric double polarisation . This is an oriented object like S3 in which the perfect symmetry around the axis is replaced with a 2-order symmetry around the axis. S4 could be used to represent carboxylic groups in their basic form.
S5: double polarisation . S5 may be used to represent objects with no symmetry axis such as amide groups.

Geometrical contructs S1 S2, S3, S4 and S5 are defined using vectors in addition to the spatial position of the chemical groups, as illustrated in figure 3.
Chemical groups, according to the invention are independent from the comparison algorithms and thus may be changed according to the user's requirements.

1.1- Local density

The parameter called "local density" D is calculated for each atom A occupying a spatial position P (figure 1) and used in the comparison process. Its purpose is to give a discriminative estimation of the burial of a said atom.
The burial of atoms may be estimated using a continuous local atomic density function. The general expression of a local density $D(x_p, y_p, z_p)$ around the position **P** is:

$$D(x_P, y_P, z_P) = \frac{\int_{-\infty}^{+\infty}\int_{-\infty}^{+\infty}\int_{-\infty}^{+\infty} w(x - x_P, y - y_P, z - z_P) \cdot m(x, y, z) \cdot dx \cdot dy \cdot dz}{\int_{-\infty}^{+\infty}\int_{-\infty}^{+\infty}\int_{-\infty}^{+\infty} w(x, y, z) \cdot dx \cdot dy \cdot dz}$$

where x, y and z are spatial coordinates, m is the density function and w is a weight function to reduce the influence of the peripheral atoms around a given one.
A spherical weight function can be used:

$$w(x, y, z) = \frac{1}{4}\left(1 - \frac{r}{r_C}\right)$$

if $r \leq r_c$, 0 otherwise
where $r$ is $\sqrt{x^2+y^2+z^2}$ and $r_c$ a critical radius. Factor [1/4] allows to make $r$ independent from $r_c$ if $m$ is constant.
The burial of a given chemical group may then be estimated by two alternative means:

a) by calculating the arithmetic mean of the local atomic densities around each atom belonging to this group.
b) by using the local atomic density for the position of its center.

1.2- Local center of mass.

For each atom with center P, a vector that indicates the exterior of the 3D atomic structure is computed.
This vector indicating the exterior of the 3D atomic structure may be represented by a density gradient.
Vector $\overrightarrow{\mathbf{CP}}$ wherein point C is the local center of mass of atom A occupying a position P may be used.
The local center of mass C(P) for point P is a point which cartesian coordinates $(x_c, y_c, z_c)$ match the following formulation:

$$\begin{cases} x_C(x_P, y_P, z_P) = \dfrac{\int_{-\infty}^{+\infty}\int_{-\infty}^{+\infty}\int_{-\infty}^{+\infty} w(x - x_P, y - y_P, z - z_P) \cdot m(x, y, z) \cdot x \cdot dx \cdot dy \cdot dz}{\int_{-\infty}^{+\infty}\int_{-\infty}^{+\infty}\int_{-\infty}^{+\infty} w(x - x_P, y - y_P, z - z_P) \cdot m(x, y, z) \cdot dx \cdot dy \cdot dz} \\ y_C(x_P, y_P, z_P) = \dfrac{\int_{-\infty}^{+\infty}\int_{-\infty}^{+\infty}\int_{-\infty}^{+\infty} w(x - x_P, y - y_P, z - z_P) \cdot m(x, y, z) \cdot y \cdot dx \cdot dy \cdot dz}{\int_{-\infty}^{+\infty}\int_{-\infty}^{+\infty}\int_{-\infty}^{+\infty} w(x - x_P, y - y_P, z - z_P) \cdot m(x, y, z) \cdot dx \cdot dy \cdot dz} \\ z_C(x_P, y_P, z_P) = \dfrac{\int_{-\infty}^{+\infty}\int_{-\infty}^{+\infty}\int_{-\infty}^{+\infty} w(x - x_P, y - y_P, z - z_P) \cdot m(x, y, z) \cdot z \cdot dx \cdot dy \cdot dz}{\int_{-\infty}^{+\infty}\int_{-\infty}^{+\infty}\int_{-\infty}^{+\infty} w(x - x_P, y - y_P, z - z_P) \cdot m(x, y, z) \cdot dx \cdot dy \cdot dz} \end{cases}$$

where x, y and z are spatial coordinates, m is the density function and w is a weight function.
The weight function w may be defined similarly to the weight function used in the local density expression defined in section 1.1.

1.3- Orientation

The orientation of each atom A occupying the position P and associated to the local center of mass C, is done by the vector $\overrightarrow{CP}$ that points towards the exterior of the 3D atomic structure. The notion of exterior depends on the weight function that has been adopted for the calculation of the center of mass.

For all kinds of molecules, using a weight function w as explicitly defined in section 1.1, a critical radius $r_c$ ranging from 3 to 50 Å, preferably from 5 to 20 Å is used.

Once defined and calculated, physico-chemical parameters that characterize the environment surrounding each atom are computed.

Every chemical group of the 3D atomic structure consists of a set of atoms as defined in said input file (figure 2). Thus for a given chemical group, the mean position *P* of these atoms, the mean position *C* of the local centers of mass and the mean local density *D* are computed and recorded.

This step reduces the representation of the 3D atomic structure by a set of chemical groups instead of atoms (figure 4A).

1.4- Further selection of chemical groups

At this stage, a selection over the chemical groups is performed (figure 4B) . The following procedures allow the user to select specific parts of the molecular structure:

a) automatic selection of most exposed chemical groups using a local density function and a threshold,
b) semi-automatic selection of chemical groups that are possibly interacting with a given set of chemical groups,
c) manual selection of subsets of chemical groups.

Step (b) is based on the definition for each chemical group of a set of points called virtual interactors.

A chemical group in a given molecule is here denoted *(P,L)* where *P* is its position and *L* is the set of points constituting the virtual interactors. A given group $(P_i, L_i)$ is said to be interacting with (*P*,*L*) if and only if there exists at least one point *Q* belonging to $L_i$ such that $Pg \leq d_{max}$ , where $d_{max}$ is an empirical threshold.

For example, two virtual interactors can be defined for aromatic rings, each of them being located symmetrically on both sides of the aromatic ring at a distance of 4 Å of its center.

**2- Cluster construction (3D substructures).**

Once constructed, sets of chemical groups, constituting 3D substructures, are selected in order to construct clusters of at least three chemical groups by setting said chemical groups whose reciprocal distances are positioned inside a sphere of radius r, where said radius is comprised between 2 to 20 Å, preferably between 2 to 8 Å.

An algorithm for finding neighboring points in constant time is used, so that the complexity of this step is proportional to the global number of chemical groups, given a definitive cluster size.

In a particular embodiment of the process to identify 3D substructures of the invention, the sets of chemical groups are selected in such a way that they comprise three chemical groups (triplets). Then, the sets of chemical groups that represent the 3D atomic structure are converted to triangles of chemical groups. In this particular embodiment of the invention, only triangles with edges smaller than a given threshold are considered.

Sets of chemical groups are oriented against the surface, for instance, in the particular embodiment of sets of three chemical groups, the orientation of a triangle (P1; P2; P3) of chemical groups is estimated, for example, by using the scalar triple product of $\overrightarrow{CP}_1$, $\overrightarrow{CP}_2$ and $\overrightarrow{CP}_3$ , wherein C is the centre of the local centres of mass of P1, P2 and P3.

Once the triangles obtained, the set of triangles representing one 3D atomic structure is converted into a graph in which

- each vertex represents one triangle;
- each edge represent the adjacency of two triangles.

Also, additional parameters may be added to this graph. For instance, the angle between the adjacent triangles is associated to each edge in the graph.

For every triangle (figure 4C) formed by three chemical groups *(P1, P2, P3)* with edges shorter than a given threshold several parameters are computed:

(1) the distances between two vertices of a triad are computed and recorded;

(2) the burial of each chemical group is estimated using the local density;

(3) the orientation of the triangle towards the rest of the 3D atomic structure is estimated by the scalar triple product of $(\overrightarrow{CP_1}, \overrightarrow{CP_2}, \overrightarrow{CP_3})$, $C$ being the local center of mass of the triad, i.e. the center of $C_1$, $C_2$ and $C_3$ which are the local centers of mass of the chemical groups located at $P_1$, $P_2$ and $P_3$. The final representation of the 3D atomic structure is obtained by connecting adjacent triangles, i.e. triangles that share exactly two chemical groups, to make a graph in which each triangle forms a vertex (figure 4D).

**3 Pairwise comparison of the 3D atomic structures.**

Once the 3D atomic structures to be compared are selected, and once the construction of clusters of chemical groups, i.e. triangles comprising triplets of chemical groups onto each one of the 3D atomic structures was done, the comparison of said structures can be generated on the fly or may be retrieved from a database (figure 5A).

Then, the process of comparison of two 3D atomic structures comprises the steps hereafter described:

3.1 <u>Searching of pairs of similar clusters of chemical groups. (i.e. triplets).</u>

The criteria for pair similarity are various and can be selected among the group consisting of:

(a) same kind of chemical groups,

(b) similar orientation of the equivalent chemical groups in the two triangles, once said triangles have been superposed, after a scoring function specific to each kind of chemical group,

(c) the same length of equivalent edges in the two triangles,

(d) the similar burial of the equivalent chemical groups in the two triangles (local density),

(e) the similar orientation of the two triangles, regarding the rest of the 3D atomic structure (scalar triple product).

3.2 <u>The calculation of a score indicating the level of similarity of clusters of said pair.</u>

Said score is then computed.

The criteria for selecting a given pair of clusters are the following:

a) every score associated to a given parameter must be above a given threshold; said threshold being either constant or dependent on the type of chemical group or the environment. A parameter has been designated to compare each chemical group, for example, aromatic group and guanidium group correspond both to a « bipolar » construction, but the first one has an « angle » parameter with a value of 60 degrees and the second one has an « angle » parameter with a value of 45 degrees.

b) the global score for the pair of clusters must be above a given threshold. Said global score combines individual scores obtained at step (a), by using a linear combination of these scores.

Thresholds for individual scores at step (a) or global score at step (b) may be also designed empirically, possibly using automated optimizations based on statistical studies.

In the particular embodiment of clusters of three chemical groups, once selected, each pair of similar triplets forms a vertex in a comparison graph.

Given two graphs of triangles, representing two 3D atomic structures $M_1$ and $M_2$, vertices $T_{1,1}$ and $T_{1,2}$ in 3D atomic structure $M_1$, and vertices $T_{2,1}$ and $T_{2,2}$ in the 3D atomic structure $M_2$, the criteria for connecting pairs ($T_{1,1}$; $T_{2,1}$) and ($T_{1,2}$; $T_{2,2}$) in the comparison graph are the following:

(a) $T_{1,1}$ and $T_{1,2}$ must be connected (adjacent) in $M_1$;

(b) $T_{2,1}$ and $T_{2,2}$ must be connected (adjacent) in $M_2$;

(c) the angle between $T_{1,1}$ and $T_{1,2}$ must be similar to the angle between $T_{2,1}$ and $T_{2,2}$

The independent subgraphs in the comparison graph for the 3D atomic structures $M_1$ and $M_2$ represent then a set of pairs of equivalent triangles corresponding to two structurally equivalent regions in 3D atomic structures $M_1$ and $M_2$.

3.3 <u>Optimization steps</u>

To optimize the obtained results, the process of the invention could further comprise one or several additional steps.

The first results conducted to sets of pairs of similar triangles. The pairs of similar triangles are converted into pairs of chemical groups. Such sets of pairs of converted chemical groups are hereafter called patches.

The patches are then refined using a selection procedure (figure 6) comprising the following steps:

1) Pairs of chemical groups within a patch may be superposed by minimizing a distance function. The distance function is the following:

$$\mathrm{dist}(g_1,g_2) = \alpha.\|pos(g_1) - pos(g_2)\|+\beta\cdot|\ D(g_1) - D(g_2)|+\gamma\cdot orient(g_1,g_2)$$

where *pos(g)* is the position of the chemical group *g* after optimal superimposition of the given set of pairs, and D *(g)* its local density; $orient(g_1,g_2)$ is the difference of orientation between chemical groups $g_1$ and $g_2$ after optimal superimposition. $\alpha$, $\beta$ and $\gamma$ are weighting coefficients that are defined on an empirical basis.

2) Several cycles with different elimination thresholds may be performed successively or in parallel by

a) calculating a score including superposition quality and the number of pairs in the patch and
b) elimination of patches having a score inferior to the current threshold.

3) Iterative steps comprising both steps of calculation and elimination may be performed to lead to a final score.

4) Finally, patches whose final score is below a given threshold are not retained.

At this step, the definitive patches are known among others. An additional parameter called atomic volume difference is computed for each patch and is used as an additional criterion for:

a) eliminating patches with low shape similarity,
b) building a more relevant score.

The purpose of the computation of the atomic volume difference is to compare the volumes of three kinds of atoms for a given patch:

$V_1$: atoms surrounding chemical groups in cluster (3D atomic substructure) extracted from $M_1$;
$V_2$: atoms surrounding chemical groups in cluster (3D atomic substructure) extracted from $M_2$;
V: atoms surrounding chemical groups in both clusters after superposition of the said clusters.

If $V_1$ and $V_2$ are similar, and if V is similar to $V_1$ and to $V_2$, then the repartition of the atoms around the selected groups is similar.

If $V_1$ and $V_2$ are similar, but V is much higher than $V_1$ or $V_2$, then the repartition of the atoms around the selected chemical groups is much different.

A score that represents atomic volume difference is derived from these calculations.

Here is an example of such a function:

$$s(v_1,v_2,v) = 1-\frac{2v - (v_1+v_2)}{v}$$

In a particular embodiment the 3D substructures of the 3D atomic structures are compared in order to obtain a list of similar 3D substructures that are associated to a score that combines several of the following criteria on an empirical basis:

a) deviation after optimal superimposition,
b) average difference of local density between chemical groups,
c) difference in the orientation of the superimposed chemical groups, using a specific scoring function for each kind of chemical group,
d) volume of the chemical groups,
e) difference in the shape of the patches by means of an atomic volume difference calculation

**4- Multiple comparison of 3D atomic structures.**

4.1 Introduction

The process for finding similarities across several molecular structures relies on the pairwise comparison of all structures. This process is schematically illustrated on figure 7. The result consists in a set of families that have the following properties:

a) a given family does not necessary concern all molecules that are being compared. Thus, this process is not influenced by the addition of a foreign molecular structure that does not share any similarity with the other molecular structures;
b) a family is a set of elements that stand for a set of chemical groups belonging to a single molecular structure;
c) each chemical group belonging to a given member of a family can be associated to a coefficient that indicates its frequency within the family, and therefore its importance in this family.

4.2 Preliminary definitions

Definition 1

A clique is a complete subgraph from a given graph.

Definition 2

For a real parameter λ in the range [0;1] , a λ-clique *C* in a given graph *G* is a subgraph such that every vertex from *C* is connected to at least λ·(|*C*|-1) vertices from *C*, where |*C*| is the cardinality of *C*.
Thus, clique is a synonym for 1-clique.

Definition 3

A maximal clique is a clique which is not a subset of any other clique.

Definition 4

A maximal λ-clique is a λ-clique which is not a subset of any other λ-clique.

4.3 Description of the process

4.3.1 Clusterization of overlapping sets of chemical groups

Let **M** be the set of molecular structures:

$$\mathbf{M}=\{M_1,\ldots,M_n\}$$

For a given structure $M_i$ comprising a set of chemical groups:

$$M_i=\{g_{i,1},\ldots,g_{i,|M_i|}\}$$

The applicant defines a comparison function that returns a set of sets of pairs (P) of chemical groups:

$$\text{comparison}(M_i,M_j)=\{P_{i,j,1},P_{i,j,2},\ldots,P_{i,j,|\text{comparison}(M_i,M_j)|}\}$$

where

$$P_{i,j,k}=\{(g_{i,\alpha_{k,1}},g_{j,\beta_{k,1}}),(g_{i,\alpha_{k,2}},g_{j,\beta_{k,2}}),\ldots,(g_{i,\alpha_{k,|P_{i,j,k}|}},g_{j,\beta_{k,|P_{i,j,k}|}})\}$$

Extraction of the chemical groups belonging to each of the structures is then operated by means of the following functions $s_1$ and $s_2$ :

$$\left| \begin{array}{l} s_1(P_{i,j,k})=\left\{g_{i,\alpha_{k,1}},g_{i,\alpha_{k,2}},\ldots,g_{i,\alpha_{k,|P_{i,j,k}|}}\right\} \\ s_2(P_{i,j,k})=\left\{g_{j,\beta_{k,1}},g_{j,\beta_{k,2}},\ldots,g_{j,\beta_{k,|P_{i,j,k}|}}\right\} \end{array} \right.$$

Then $\mathbf{P}_i$ is defined as follows:

$$\mathbf{P}_i=\{s_1(P_{i,j,k})\mid 1\leq j\leq m \text{ and } P_{i,j,k}\in\text{comparison}(M_i,M_j)\}$$

Then, for a given molecule $M_i$, the method of the invention comprises the construction of a graph $G_i$ consisting of:

a) vertices that match the elements of $P_i$,
b) edges that connect any vertices $u$ and $v$ sufficiently overlapping according to a given predicate such as the following:

$$\frac{|u\cap v|}{\max(|u|,|v|)}\geq\text{overlap}$$

In this particular embodiment of the invention, overlap is a real number within the range 0 and 1,preferably between 0,5 and 1 and more preferably 0,7.

The maximal cliques from $G_i$ are denoted as follows:

$$\text{maximal-cliques}(G_i)=\{V_{i,1},V_{i,2},\ldots,V_{i,v_i}\}$$

4.3.2 Extraction of families

In order to operate the extraction of the families of similar clusters of chemical groups that are found similar in several molecular structures, the applicant has been defined the following function :

$$\text{families}(\mathbf{M})=\text{maximal-}\lambda\text{-cliques}(H(\mathbf{M}))$$

wherein $H(\mathbf{M})$ is the graph such that:

a)

$$\texttt{vertices} = \bigcup_{i=1}^{m}\text{maximal-cliques}(\mathbf{P}_i)$$

b) there is an edge connecting vertices $V_{i,a}$ and $V_{j,b}$ if and only if:

$$\exists k \text{ such that } \begin{cases} s_1(P_{i,j,k}) \in V_{i,a} \\ s_2(P_{i,j,k}) \in V_{j,b} \end{cases}$$

Note that a cluster *V* of chemical groups is a set of overlapping sets of chemical groups. *V* can be converted into a set $\{(g_1,w_1),(g_2,w_2),...\}$ where each chemical group $g_i$ is associated to a weight $w_i$ that may range from 1 to $|V|$ and is the number of occurrences of this chemical group in the elements of *V*.

4.4 Algorithms

The problem of finding the maximum clique in an arbitrary graph, i.e. the maximal clique of maximum cardinality, is known to be *NP*-complete. Therefore, the problem of getting all maximal cliques and the larger problem of getting all maximal λ-cliques are also *NP*-complete.

However, this has not be proven for the particular cases that are considered here. The algorithm that is used for finding all maximal λ-cliques is a clustering algorithm: subgraphs are initially constituted by individual vertices and are extended using neighbor vertices under the condition that the resulting subgraph is still a λ-clique. Redundant subgraphs are removed during the process.

The applicant found that the value of 0.7 is a suitable value for both overlap and parameters in the multiple comparison process.

**5 - APPLICATION: DATABASE DESIGN -SCREENING-**

5.1 Design of databases for efficient screening

Database containing 3D atomic substructures of 3D structures may be preformatted to allow a quick comparison of one of its members to any other 3D structure preformatted.

The design of said databases can be applied to any kind of 3D atomic structures such as proteins, nucleic acids or other natural and artificial polymers, but also non-polymeric atomic 3D structures.

The database may comprise complete 3D atomic substructures but also fragments thereof.

In particular the database may comprise particular fragments of 3D atomic structures implicated in biological processes, such as enzymatic processes, reversible or irreversible binding of a class of molecules, sensitivity to a particular physico-chemical environment, energy conversion, self modification, antigenicity, modification of the intensity of a biological process.

The fragments of 3D atomic substructures to be included in the database may also be determined automatically, by selection of chemical groups that interact with a ligand which is present in the 3D atomic structure or predicted by biochemical experiences.

As ligand one may understand a 3D atomic structure, whatever is its nature, such as a peptide or an oligonucleotide, able to bind to another molecular partner, as a receptor, an antibody, a co-factor.

The selection of the sites of interaction between the 3D atomic structure and the ligand may be made by characterizing positioning around each chemical group included in essential regions of the 3D atomic structure.

Then chemical groups which surround the ligand are selected.

More precisely, the present invention relates to a process to identify similar 3D substructures onto 3D atomic structures having a plurality of individual atoms, performed with the aid of a programmed computer comprising the steps of:

a) attributing to each individual atom of said 3D atomic structure a structural parameter combining its atomic local density D, its local center of mass C and its orientation in relation with its position P.
b) constructing chemical groups by setting individual atoms having similar structural parameters,
c) constructing clusters of at least three chemical groups by setting said chemical groups whose reciprocal distances are constrained,
d) comparing clusters constructed at step (c) and identifying said clusters sharing similar 3D structures.

The atomic local density D of step (a) may be calculated for each atom A, on basis to its spatial position P defined by its coordinates $(x_p, y_p, z_p)$ as a function of its density m, modulated by a weight function w, by means of the function:

$$D(x_P, y_P, z_P) = \frac{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz}{\int_{-\infty}^{+\infty}\int_{-\infty}^{+\infty}\int_{-\infty}^{+\infty} w(x,y,z)\cdot dx\cdot dy\cdot dz}$$

And the weight function w is preferably calculated as a spherical function

$$w(x,y,z) = \frac{1}{4}\left(1-\frac{r}{r_c}\right)$$

if $r \le r_c$, 0 otherwise

wherein $r$ is $\sqrt{x^2+y^2+z^2}$, $r_c$ a critical radius and the factor [1/4] allows to make $r$ independent from $r_c$ if m is constant.

The local center of mass C(P), for a given atom A occupying a position P which cartesian coordinates ($x_c$, $y_c$, $z_c$) preferably match the following formulation:

$$\begin{cases} x_C(x_P, y_P, z_P) = \dfrac{\int_{-\infty}^{+\infty}\int_{-\infty}^{+\infty}\int_{-\infty}^{+\infty} w(x-x_P, y-y_P, z-z_P)\cdot m(x,y,z)\cdot x\cdot dx\cdot dy\cdot dz}{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz} \\ y_C(x_P, y_P, z_P) = \dfrac{\int_{-\infty}^{+\infty}\int_{-\infty}^{+\infty}\int_{-\infty}^{+\infty} w(x-x_P, y-y_P, z-z_P)\cdot m(x,y,z)\cdot y\cdot dx\cdot dy\cdot dz}{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz} \\ z_C(x_P, y_P, z_P) = \dfrac{\int_{-\infty}^{+\infty}\int_{-\infty}^{+\infty}\int_{-\infty}^{+\infty} w(x-x_P, y-y_P, z-z_P)\cdot m(x,y,z)\cdot z\cdot dx\cdot dy\cdot dz}{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz} \end{cases}$$

wherein x, y and z are spatial coordinates, m is the density function and w is a weight function.

In a particular embodiment of the process of the invention said weight function w is a spherical function:

$$w(x,y,z) = \frac{1}{4}\left(1-\frac{r}{r_c}\right)$$

if $r \le r_c$, 0 otherwise

wherein $r$ is $\sqrt{x^2+y^2+z^2}$, $r_c$ a critical radius and the factor [1/4] allows to make $r$ independent from $r_c$ if m is constant.

Also, the orientation of each atom A occupying a position P and having a local center of mass C(P) at step (a) of the process, may be preferably calculated by means of a density gradient represented by a vector $\overrightarrow{CP}$ .

In a preferred embodiments of this process to identify similar 3D substructures, the reciprocal distances between chemical groups selected in step (c) are comprised between 2 to 20 Å, preferably between 5 to 12 Å.

Also the construction of clusters at step (c) comprises orientation of said clusters against the 3D atomic structure and preferably this orientation is operated by means of a scalar triple product of three vectors $\overrightarrow{CP_i}$ , $\overrightarrow{CP_j}$ , $\overrightarrow{CP_k}$ wherein C is the center of the local centers of mass of each chemical group and $P_i$, Pj, $P_k$ are three distinct points in the cluster.

The process to identify similar 3D substructures is particular useful to construct clusters of chemical groups chat can be are further stored and classified in a database.

The comparison of a given pair of clusters at step (d) of a such process to identify similar 3D substructures comprises the identification of at least one structural similarity selected from the group consisting of:

(a) same chemical groups and similar orientation,

(b) similar length of reciprocal distances between chemical groups,
(c) similar local density of the chemical groups
(d) similar orientation of the constructed clusters

Furthermore, after identification of at least two structural similarities, the process comprises the calculation of a global score , that may be calculated as a function combining several parameters indicating the similarity of said clusters, said parameters being selected among the group consisting of :

- volume of chemical groups,
- scarcity of the chemical groups,
- quality of the superposition with respect to the standard deviation,
- quality of the superposition with respect to de orientation of the chemical groups,
- resemblance of the atomic environment.

In a particular embodiment, the resemblance between atomic environment comprises the calculation of a score by comparing volumes of atoms around each converted pair of chemical groups and a such score is calculated by means of the function:

$$s(v_1,v_2,v) = 1-\frac{2v - (v_1+v_2)}{v}$$

wherein:

$V_1$ is the volume of atoms surrounding chemical groups in cluster (3D atomic substructure) extracted from atomic structure $M_1$;
$V_2$ is the volume of atoms surrounding chemical groups in cluster (3D atomic substructure) extracted from atomic structure $M_2$;V is the volume of atoms surrounding chemical groups in both clusters after superposition of the said clusters.

Also, in a particular embodiment, the process to identify similar 3D substructures of the present invention may comprise before the step (c) a further step comprising the restriction of constructed chemical groups.
Said restriction of constructed chemical groups may be operated by means of an additional step (f) comprising the selection of chemical groups at locations where the local atomic density is below a definite threshold.
In another particular embodiment said restriction may be operated by an additional step (g) wherein the restriction of constructed chemical groups is operated by means of a selection among :

- automatic selection of most exposed chemical groups using a local density function and a threshold,
- semi-automatic selection of chemical groups that are interacting with a given set of chemical groups,
- manual selection of subsets of chemical groups.

The process to identify similar 3D substructures of the present invention may be performed by applying a refinement step comprising :

i) converting the pairs of clusters identified at step (d) to pairs of chemical groups,
ii) minimizing the reciprocal distances between converted pairs of chemical groups by means of a distance function.

Eventually, the steps (i) and (ii) are iteratively repeated using variable selection thresholds.
In particular embodiments, when said refined step is operated, the reciprocal distances between converted pairs of chemical groups may be calculated by means of the function:

$$dist(g_1,g_2) = \alpha\cdot\|pos(g_1) - pos(g_2)\| + \beta|D(g_1) - D(g_2)| + \gamma\cdot orient(g_1 ,g_2)$$

wherein *pos(g)* is the position of the chemical group *g* after optimal superimposition of the given set of converted pairs, *D(g)* its local density, $orient(g_1,g_2)$ is the difference of orientation between chemical groups $g_1$ and $g_2$ after optimal superimposition, and $\alpha$, $\beta$ and $\gamma$ are normalising coefficients that are calculated such that the average value of each term is 1/3, on the basis of a statistical set of pairs of similar chemical groups.

The process to identify 3D similar substructures according invention may further comprise a step (e) of clustering pairs of clusters identified at step (d) into a larger pair of clusters sharing similar 3D structures.

The 3D atomic structure having a plurality of individual atoms is a covalent or weak assembly of at least one molecule selected among the group comprising: natural and artificial proteins, oligopeptides, polypeptides, nucleic acids, natural and artificial oligonucleotides, natural and artificial oligosaccharides and polysaccharides, glycoproteins, lipoproteins, lipids, ions, water, natural and synthetic polymers, non polymeric structures, natural and artificial inorganic molecules.

In particular embodiments the 3D atomic substructure to be identified onto 3D atomic structures having a plurality of individual atoms is a functional site.

Said functional site may be selected among the group consisting of: enzymatic active sites, sites of reversible or irreversible binding of specific classes of molecules, sites sensitive to physico-chemical changes in the environment, chemical groups involved in energy conversion, self modification locations, antigenic parts of a molecule, mimetic sites, consensus sites, highly variable sites, sites necessary for initiating or interrupting a biological pathway, sites with particular physico-chemical properties, sites with particular chemical composition, protein taxons, immunoglobulin domains, DNA consensus sequences, gene expression signals, promoter elements, RNA processing signals, translational initiation sites, recognition motifs of a large variety of sequence-specific DNA-binding proteins, protein and nucleic acid compositional domains, glutamine-rich activation domains, CpG island, interaction site between a protein and a ligand, functional sugar binding site.

Also, the 3D atomic substructures to be identified onto 3D atomic structures having a plurality of individual atoms may be 3D structural sites issued from combinatorial, or conventional screening.

The invention relates also to a process to predict functional sites onto 3D atomic structures comprising the identification of 3D atomic substructures by means of a process according to the invention, further comprising the correlation of said identified 3D atomic substructures with a known biological or chemical function.

Another object of the invention is a process to identify similar 3D atomic substructures A and B according to the invention, further comprising calculation of average orientation of said 3D atomic substructures A and B with respect to the orientation of their individual atoms and a visual representation of said A and B 3D atomic substructures, wherein said visual representation is operated by means of graphs projections matching the following conditions:

a) the average orientation of said 3D A atomic structure is orthogonal to the projection plane;
b) said substructure B is optimally superimposed to said substructure A.

The invention will be illustrated hereafter with some obtained experimental results and in view of figures 1 to 12 in appendix wherein:

Figures 1 to 7 illustrate major steps of the process of comparison of 3D structures according to the invention.

- Figure 1 shows the local density computation around a given atom A with a plot representing the weight function.
- Figure 2 illustrates an example of two chemical groups as currently defined.
- Figure 3 represents the different vectors useful to represent geometric information associated to each chemical group and related to their spatial orientation.
- Figure 4 illustrates the reduction of atoms to chemical groups (A), followed by the selection of chemical groups according to the user's will (B). This figure reflects the particular embodiment in which three chemical groups are selected to form triangles graphs (C), followed by a computation of parameters associated to each triangle (D). Letters are those used in the text: $P_1$ , $P_2$ and $P_3$ represent the position of the chemical groups, $C_1$, $C_2$ and $C_3$ represent the local centers of mass associated to the chemical groups, *P* and *C* are the centers of these points.
- Figure 5 corresponds to an input of graphs from a database (A) and illustrates the main comparison step (B) .
- Figure 6 illustrates an optional refinement step.
- Figure 7 illustrates the process of obtaining families of substructures from an arbitrary number of structures, by starting using pairwise comparison results. The example shows 3 molecules denoted by M1, M2 and M3 as in the text.

Figures 9 to 11 illustrate the screening results and information about the legume lectin family.

Figures 8 and 9 illustrate the comparison of serine proteases: subtilisin, 1SBC structure vs. chymotrypsin, 1AFQ structure.

- Figure 8 is a schematic view of the sequences of both functional forms of the serine proteases with highlighting of the catalytic triad residues.
- Figure 9 shows the computer file result obtained by applying the process of the invention to proteases 3D structures.
- Figure 10 illustrates results obtained by the process of comparison of the invention ; no incorrect patch was returned.

- Figure 11 is a view of the chemical groups defining the sugar-binding site in the peanut lectin structure 2PEL.
- Figure 12 illustrates essential aminoacids for sugar binding in concanavalin A before and after demetallization; superposition of α-carbons with 0.9 Å RMSD of 1DQ1 (native form) and 1DQ2 (apoprotein).

Example 1

Structural similarities among Serine proteases.

Subtilisin and γ-chymotrypsin are endoproteases sharing a similar catalytic site: both mechanisms use catalytic triad formed by an aspartate, an histidine and a serine. These proteins do not share either a sequence similarity nor a similar fold in spite of their highly similar active sites. Figure 8 shows that the position of these residues has neither the same position nor the same order within the sequence, making it irrelevant to align their sequences. Structures 1SBC of subtilisin and 1AFQ of γ-chymotrypsin have been compared by the process of comparison of the invention. The result file is shown on figure 9 and displays one similar region that consists of the catalytic triad (Asp32/Asp102 His64/His57, Ser221/Ser195) represented but four chemical groups, plus a glycine (G1y127/G1y216) which is also known to play a role in the protease activity [18].

Example 2

Structural similarities between Legume lectins.

The structural family of legume lectins is represented by 106 structures publicly available in the PDB.

Many of them are functional lectins, i.e. proteins that bind oligosaccharides non-covalently, but some of them have lost the capability to bind sugar at this site in spite of their overall sequential and structural similarity (see [19] for a full review on lectins). Proteins without native sugar-binding ability are arcelin and α-amylases inhibitors (4 structures). 7 structures are available of demetallized lectins, i.e. lectins whose site has been deprived of Ca 2+ and Zn 2+ . For example, 1DQ1 and 1DQ2 are 2 structures of concanavalin A in both native and demetallized forms: though their sequences are identical and their backbone have an RMSD of 0.9 Å for α-carbons, only the first form binds a sugar 3D atomic structure.

Structure 2PEL of the peanut lectin has been used to represent a functional lectin: its site of interaction with lactose has been selected and compared to every structure within the family. More precisely, all groups that have at least one atom closer than 4 Å to any atom of the ligand were selected. Thus, 10 chemical groups covering 9 aminoacids were retained (figure 11). The result of these comparisons is summarized on figure 10. Among all structures, 91 proteins showed at least one similar patch. All of these patches were sugar binding sites from functional proteins. No patch was detected among the 11 proteins missing the sugar-binding function. Thus, only 4 functional sites were not detected, and no false positive was obtained. Local conformational changes at the binding site explain the lost of activity in the case of demetallized lectins as shown on figure 12.

With the example of legume lectins, the applicant shows that the process to identify 3D substructures of the present invention excludes non-functional lectins by comparing them to a functional sugar binding site in spite of a high degree of similarity in sequence and in main chain architecture. This indicates that something like structural flexibility is taken into account by said process. It is sensitive enough to detect local conformation changes that are correlated with a loss of function. The process to identify 3D substructures of the present invention is also flexible enough to ignore minor changes like those depending on the presence or absence of the ligand. Only 4% of the functional lectins were not detected.

CONCLUSION

The capabilities of this tool are illustrated and validated across two extreme kinds of biological problems: (1) the case of convergent evolution, in which proteins do not share any sequential nor fold similarities but share a common biochemical activity, will be illustrated by two unrelated serine proteases; (2) the case of divergent evolution and loss of function due to minor modifications in the protein structure will be illustrated by the analysis of the legume lectins family.

The main advantages of this process reside in the fact that it :

(1) considers in a single process all relevant information provided by the protein structure, regarding protein function in its broadest sense;
(2) uses representations and strategies that fit the intuitive models for chemical interactions;

(3) chooses algorithmic strategies that allow to search the whole PDB for a site in less than one day;
(4) makes the software customizable at run time.

The process of comparison of 3D atomic structures according to the invention allows the detection of 3D structural similarities in 3D atomic structures. The applicant shows that 3D structural similarities are correctly detected in serine proteases that are differently folded and have unrelated sequences: Dali [20] finds no similarity between these structures and it is not possible to propose a valid sequence alignment due to the inversion of catalytic residues in the sequence.

In this case, the structural similarity that is automatically detected by the process of the invention corresponds to a common biochemical function and an identical catalytic mechanism.

In other cases, the applicant compares structures of proteins which are known to act as competitors in a biological process that is understood with more or less precision: enzymatic catalysis, affinity for a ligand, disruption or activation of biochemical pathways, immunological cross-reactivity, inhibition of cell adhesion, etc.

The use of chemical groups to represent elementary bricks instead of aminoacids to understand molecular functions is essential but not possible if only the sequence of the protein is known.

Aminoacids are composed of several critical groups that may or may not be important depending on the structural context (table 1). Knowing the 3D structure of proteins allows to model proteins with chemical groups, covalent bonds and other interactions independently from the concept of aminoacid.

The choice of using triplets of chemical groups as the basic information for the comparison has been made for several reasons:

(1) a triangle may be associated to a number of parameters that ensures that a given triangle contains an amount of information that makes it much more rare than a single group represented by a point. It stands for a minimal representation of a local environment, including an oriented plan;
(2) a specific biological function is rarely provided by only one or two chemical groups;
(3) the basic information that consists of the chemical group type and its position is kept along all comparison steps.
(4) adjacent triangles are easy to cluster in order to represent larger regions of 3D atomic structures;

A limit to the representation of a 3D atomic structure by a single graph of 3D located objects (such as points or triangles) is the difficulty to mix well-located and numerous objects (such as hydrogen bonds) with less located but sparser objects (such as clusters of 3 positively charged aminoacids).

The burial of atoms is not estimated using an accessible surface area (ASA) calculation, but a notion of local atomic density. In analogy to immerged bodies, the ASA would correspond to the emerged part of a floating body and be null for any object under the surface, whereas the density calculation is a measure of the depth of any object, even non-floating ones. Figure 12 shows that aspartate in the catalytic triad of serine proteases is almost completely buried, suggesting that crucial residues may be essential for protein function, and this even if they lie below the surface of the 3D atomic structure. This kind of depth estimation is also essential for providing a vector that is roughly orthogonal to the molecular surface: these vectors are used to estimate the angle formed between a given triplet of chemical groups and the surface.

As the process may be used to perform a large number of comparisons, especially when one of the compared elements is a small site, the following useful strategies may be used:

- compare two protein structures that present a poorly understood functional analogy;
- screen the PDB for a given site;
- screen a database of functional 3D sites with a newly determined structure.

This process is essentially relevant to annotate newly determined structures from structural genomics approaches. It would require to build a valid database of sites. Even this work can be partially automated by considering as a site any set of chemical groups that interact with a ligand in the PDB.

References

1. Altschul, S.F., et al., *Gapped BLAST and PSI-BLAST: a new generation of protein database search programs.* Nucleic Acids Res, 1997. 25(17): p. 3389-402.
2. Pearson, W.R., *Rapid and sensitive sequence comparison with FASTP and FASTA.* Methods Enzymol, 1990. 183: p. 63-98.

3. Bairoch, A., *PROSITE: a dictionary of sites and patterns in proteins.* Nucleic Acids Res, 1991. 19 Suppl : p. 2241-5.
4. Gribskov, M., A.D. McLachlan, and D. Eisenberg, *Profile analysis: detection of distantly related proteins.* Proc Natl Acad Sci U S A, 1987. 84(13): p. 4355-8.
5. Blanchet, C., *Logiciel MPSA et ressources bioinformatiques client-serveur Web dedies a l'analyse de sequences de protéine.* 1999, Université Claude Bernard, Lyon 1: Lyon, France.
6. Thompson, J.D., D.G. Higgins, and T.J. Gibson, *CLUSTAL W: improving the sensitivity of progressive multiple sequence alignment through sequence weighting, position-specific gap penalties and weight matrix choice.* Nucleic Acids Res, 1994. 22(22): p. 4673-80.
7. Holm, L. and C. Sander, *Protein structure comparison by alignment of distance matrices.* J. Mol. Biol., 1993. 233: p. 123-138.
8. Holm, L. and C. Sander, *Touring protein fold space with Dali/FSSP.* Nucleic Acids Res, 1998. 26(1): p. 316-9.
9. Fischer, D., et al., *An efficient automated computer vision based technique for detection of three dimensional structural motifs in proteins.* J Biomol Struct Dyn, 1992. 9(4): p. 769-89.
10. Lin, S.L., et al., *Molecular surface representations by sparse critical points.* Proteins, 1994. 18(1): p. 94- 101.
11. Sandak, B., R. Nussinov, and H.J. Wolfson, *An automated computer vision and robotics-based technique for 3-D flexible biomolecular docking and matching.* Comput Appl Biosci, 1995. 11(1): p. 87-99.
12. Preissner, R., A. Goede, and C. Frommel, *Dictionary of interfaces in proteins (DIP). Data bank of complementary molecular surface patches.* J Mol Biol, 1998. 280(3): p. 535-50.
13. Preissner, R., A. Goede, and C. Frommel, Homonyms and synonyms in the *Dictionary* of Interfaces in *Proteins (DIP).* Bioinformatics, 1999. 15(10): p. 832-6.
14. Katchalski-Katzir, E., et al., *Molecular surface recognition: determination of geometric fit between proteins and their ligands by correlation techniques.* Proc Natl Acad Sci U S A, 1992. 89(6): p. 2195-9.
15. Karlin, S. and Z.Y. Zhu, *Characterizations of diverse residue clusters in protein three-dimensional structures.* Proc Natl Acad Sci U S A, 1996. 93(16): p. 8344-9.
16. Wei, L. and R.B. Altman, *Recognizing protein binding sites using statistical descriptions of their 3D environments.* Pac Symp Biocomput, 1998: p. 497-508.
17. Taroni, C., S. Jones, and J.M. Thornton, *Analysis and prediction of carbohydrate binding sites.* Protein Eng, 2000. 13(2): p. 89-98.
18. Voet, D. and J. Voet, *Biochemistry.* 1997, Wiley and Sons. p. 389-400.
19. Lis, H. and N. Sharon, *Lectins: carbohydrate-specific proteins that mediate cellular recognition.* Chem. Rev., 1998. 98: p. 637-674.
20. Holm, L. and C. Sander, *Dali/FSSP classification of three-dimensional protein folds.* Nucleic Acids Res, 1997. 25(1): p. 231-4.

## Claims

**1.** A process to identify similar 3D substructures onto 3D atomic structures having a plurality of individual atoms, performed with the aid of a programmed computer comprising the steps of:

a) attributing to each individual atom of said 3D atomic structure a structural parameter combining its atomic local density D, its local center of mass C and its orientation in relation with its position P.
b) constructing chemical groups by setting individual atoms having similar structural parameters,
c) constructing clusters of at least three chemical groups by setting said chemical groups whose reciprocal distances are constrained,
d) comparing clusters constructed at step (c) and identifying said clusters sharing similar 3D structures.

**2.** A process to identify similar 3D substructures according to claim 1 wherein the atomic local density D of step (a) is calculated for each atom A, on basis to its spatial position P defined by its coordinates ($x_p$, $y_p$, $z_p$) as a function of its density m, modulated by a weight function w, by means of the function:

$$D(x_P, y_P, z_P) = \frac{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz}{\int_{-\infty}^{+\infty}\int_{-\infty}^{+\infty}\int_{-\infty}^{+\infty} w(x,y,z)\cdot dx\cdot dy\cdot dz}$$

**3.** A process to identify similar 3D substructures according to claim 2 wherein the weight function w is a spherical function

$$w(x,y,z) = \frac{1}{4}\left(1-\frac{r}{r_C}\right)$$

if $r \le r_C$ , 0 otherwise

where r is $\sqrt{x^2+y^2+z^2}$, $r_C$ a critical radius and the factor [1/4] allows to make *r* independent from $r_C$ if *m* is constant.

**4.** A process to identify similar 3D substructures according to anyone of claims 1 to 3 wherein the local center of mass C(P), for a given atom A occupying a position P is calculated as a point which cartesian coordinates ($x_C$, $y_c$, $z_c$) match the following formulation:

$$\begin{cases} x_C(x_P, y_P, z_P) = \dfrac{\int_{-\infty}^{+\infty}\int_{-\infty}^{+\infty}\int_{-\infty}^{+\infty} w(x-x_P, y-y_P, z-z_P)\cdot m(x,y,z)\cdot x\cdot dx\cdot dy\cdot dz}{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz} \\ y_C(x_P, y_P, z_P) = \dfrac{\int_{-\infty}^{+\infty}\int_{-\infty}^{+\infty}\int_{-\infty}^{+\infty} w(x-x_P, y-y_P, z-z_P)\cdot m(x,y,z)\cdot y\cdot dx\cdot dy\cdot dz}{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz} \\ z_C(x_P, y_P, z_P) = \dfrac{\int_{-\infty}^{+\infty}\int_{-\infty}^{+\infty}\int_{-\infty}^{+\infty} w(x-x_P, y-y_P, z-z_P)\cdot m(x,y,z)\cdot z\cdot dx\cdot dy\cdot dz}{\int_{-\infty}^{+\infty}\int_{-\infty}^{+\infty}\int_{-\infty}^{+\infty} w(x-x_P, y-y_P, z-z_P)\cdot m(x,y,z)\cdot dx\cdot dy\cdot dz} \end{cases}$$

where x, y and z are spatial coordinates, m is the density function and w is a weight function.

**5.** A process to identify similar 3D substructures according to claim 4 wherein the weight function w is a spherical function:

$$w(x,y,z) = \frac{1}{4}\left(1-\frac{r}{r_C}\right)$$

if $r \le r_C$ , 0 otherwise

where *r* is $\sqrt{x^2+y^2+z^2}$ , $r_c$ a critical radius and the factor [1/4] allows to make *r* independent from $r_C$ if *m* is constant.

**6.** A process to identify similar 3D substructures according to claims 1 to 5 wherein at step (a) the orientation of each atom A occupying a position P and having a local center of mass C(P) is calculated by means of a density gradient represented by a vector $\overrightarrow{CP}$ .

**7.** A process to identify similar 3D substructures according to anyone of previous claims wherein the reciprocal distances between chemical groups in step (c) are comprised between 2 to 20 Å, preferably between 5 to 12 Å.

**8.** A process to identify similar 3D substructures according to anyone of previous claims wherein constructing clusters at step (c) comprises orientation of said clusters against the 3D atomic structure.

**9.** A process to identify similar 3D substructures according to claim 8 wherein the orientation of the constructed cluster against the 3D atomic structure is operated by means of a scalar triple product of three vectors $\overrightarrow{CP_i}$ , $\overrightarrow{CP_j}$ , $\overrightarrow{CP_k}$ wherein C is the center of the local centers of mass of each chemical group and $P_i$, $P_j$, $P_k$ are three distinct points in the cluster.

**10.** A process to identify similar 3D substructures according to anyone of previous claims wherein clusters of chemical groups are further stored in a database.

**11.** A process to identify similar 3D substructures according to anyone of previous claims wherein the comparison of a given pair of clusters at step (d) comprises the identification of at least one structural similarity selected from the group consisting of:

(a) same chemical groups and similar orientation,
(b) similar length of reciprocal distances between chemical groups,
(c) similar local density of the chemical groups
(d) similar orientation of the constructed clusters

**12.** A process to identify similar 3D substructures according to claim 11 wherein after identification of at least two structural similarities, a global score is calculated as a function combining several parameters indicating the similarity of said clusters, said parameters being selected among the group consisting of :

- volume of chemical groups,
- scarcity of the chemical groups,
- quality of the superposition with respect to the standard deviation,
- quality of the superposition with respect to de orientation of the chemical groups,
- resemblance of the atomic environment.

**13.** A process to identify similar 3D substructures according to claim 12 wherein the resemblance between atomic environment comprises the calculation of a score by comparing volumes of atoms around each converted pair of chemical groups.

**14.** A process to identify similar 3D substructures according to claim 13 wherein the score is calculated by means of the function:

$$s(v_1,v_2,v) = 1-\frac{2v - (v_1+v_2)}{v}$$

wherein:

$V_1$ is the volume of atoms surrounding chemical groups in cluster (3D atomic substructure) extracted from atomic structure $M_1$;
$V_2$ is the volume of atoms surrounding chemical groups in cluster (3D atomic substructure) extracted from atomic structure $M_2$;
V is the volume of atoms surrounding chemical groups in both clusters after superposition of the said clusters.

**15.** A process to identify similar 3D substructures according to anyone of previous claims wherein before the step (c) a further step (f) comprising the restriction of constructed chemical groups is operated by means of selection of chemical groups at locations where the local atomic density is below a definite threshold.

**16.** A process to identify similar 3D substructures according to anyone of claims 1 to 14 wherein before of step (c) a further step (g) comprising the restriction of constructed chemical groups is operated by means of a selection

among :

- automatic selection of most exposed chemical groups using a local density function and a threshold,
- semi-automatic selection of chemical groups that are interacting with a given set of chemical groups,
- manual selection of subsets of chemical groups.

**17.** A process to identify similar 3D substructures according to any claims 1 to 16 further comprising a refinement step comprising :

i) converting the pairs of clusters identified at step (d) to pairs of chemical groups,
ii) minimizing the reciprocal distances between converted pairs of chemical groups by means of a distance function.

**18.** A process to identify similar 3D substructures according to claim 17 wherein the steps (i) and (ii) are iteratively repeated using variable selection thresholds.

**19.** A process to identify similar 3D substructures according to claim 17 wherein the reciprocal distances between converted pairs of chemical groups are calculated by means of the function:

$$dist(g_1,g_2) = \alpha \parallel pos(g_1) - pos(g_2)\parallel + \beta \cdot |D(g_1) - D(g_2)| + \gamma . orient(g_1 ,g_2)$$

wherein *pos(g)* is the position of the chemical group *g* after optimal superimposition of the given set of converted pairs, *D (g)* its local density, $orient(g_1,g_2)$ is the difference of orientation between chemical groups $g_1$ and $g_2$ after optimal superimposition, and $\alpha$, $\beta$ and $\gamma$ are normalising coefficients that are calculated such that the average value of each term is 1/3, on the basis of a statistical set of pairs of similar chemical groups.

**20.** A process to identify 3D similar substructures according to any claim 1 to 19 further comprising a step (e) of clustering pairs of clusters identified at step (d) into a larger pair of clusters sharing similar 3D structures.

**21.** A process to identify similar 3D substructures according to anyone of previous claims, wherein the 3D atomic structure having a plurality of individual atoms is a covalent or weak assembly of at least one molecule selected among the group comprising: natural and artificial proteins, oligopeptides, polypeptides, nucleic acids, natural and artificial oligonucleotides, natural and artificial oligosaccharides and polysaccharides, glycoproteins, lipoproteins, lipids, ions, water, natural and synthetic polymers, non polymeric structures, natural and artificial inorganic molecules.

**22.** A process to identify 3D substructures according to claims 1 to 21 wherein the 3D atomic substructure is a functional site.

**23.** A process to identify 3D substructures according to claim 22 wherein the functional site is selected among the group consisting of: enzymatic active sites, sites of reversible or irreversible binding of specific classes of molecules, sites sensitive to physico-chemical changes in the environment, chemical groups involved in energy conversion, self modification locations, antigenic parts of a molecule, mimetic sites, consensus sites, highly variable sites, sites necessary for initiating or interrupting a biological pathway, sites with particular physico-chemical properties, sites with particular chemical composition, protein taxons, immunoglobulin domains, DNA consensus sequences, gene expression signals, promoter elements, RNA processing signals, translational initiation sites, recognition motifs of a large variety of sequence-specific DNA-binding proteins, protein and nucleic acid compositional domains, glutamine-rich activation domains, CpG island, interaction site between a protein and a ligand, functional sugar binding site.

**24.** A process to identify similar 3D substructures according to claims 1 to 23 wherein the 3D atomic substructures are 3D structural sites issued from combinatorial, or conventional screening.

**25.** A process to predict functional sites onto 3D atomic structures comprising the identification of 3D atomic substructures by means of a process according to anyone previous claims, further comprising the correlation of said identified 3D atomic substructures with a known biological or chemical function.

**26.** A process to identify similar 3D atomic substructures A and B according to any claim 1 to 24 further comprising calculation of average orientation of said 3D atomic substructures A and B with respect to the orientation of their individual atoms and a visual representation of said A and B 3D atomic substructures, wherein said visual representation is operated by means of graphs projections matching the following conditions:

a) the average orientation of said 3D A atomic structure is orthogonal to the projection plane;
b) said substructure B is optimally superimposed to said substructure A.

Figure 1

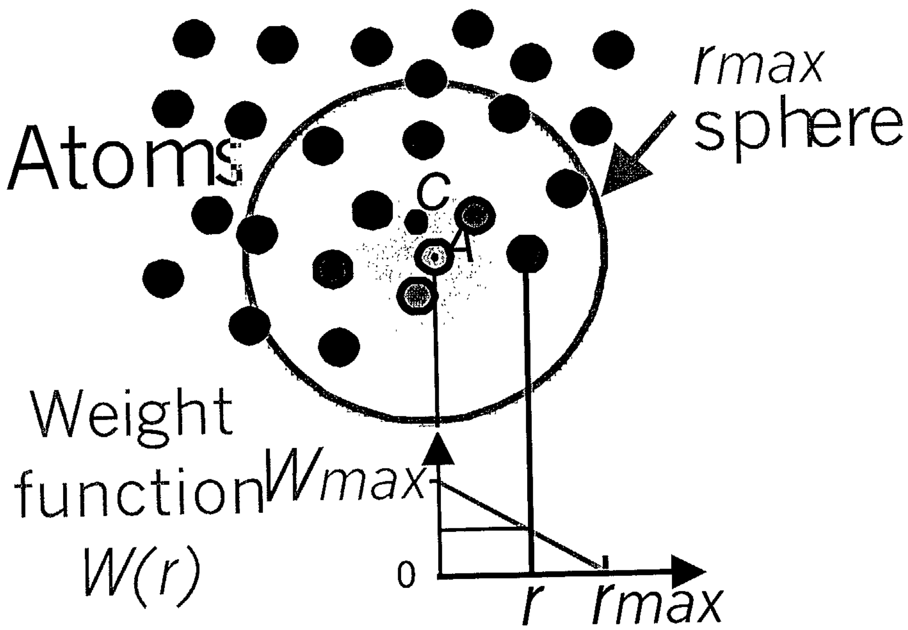

Figure 2

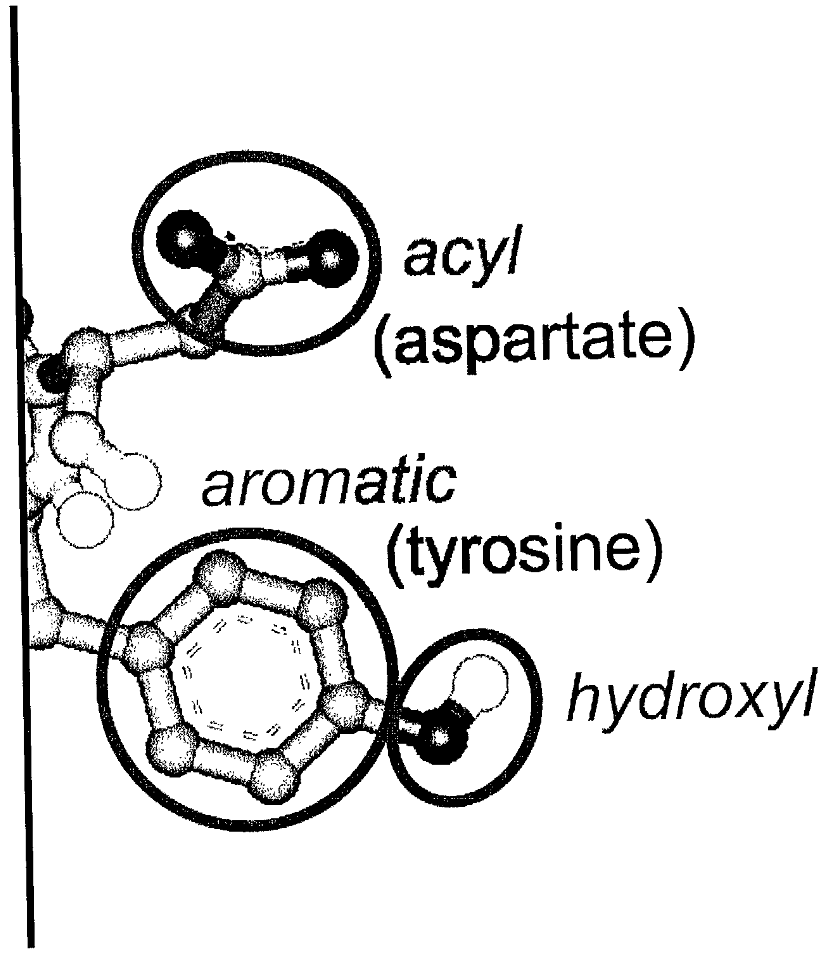

Figure 3

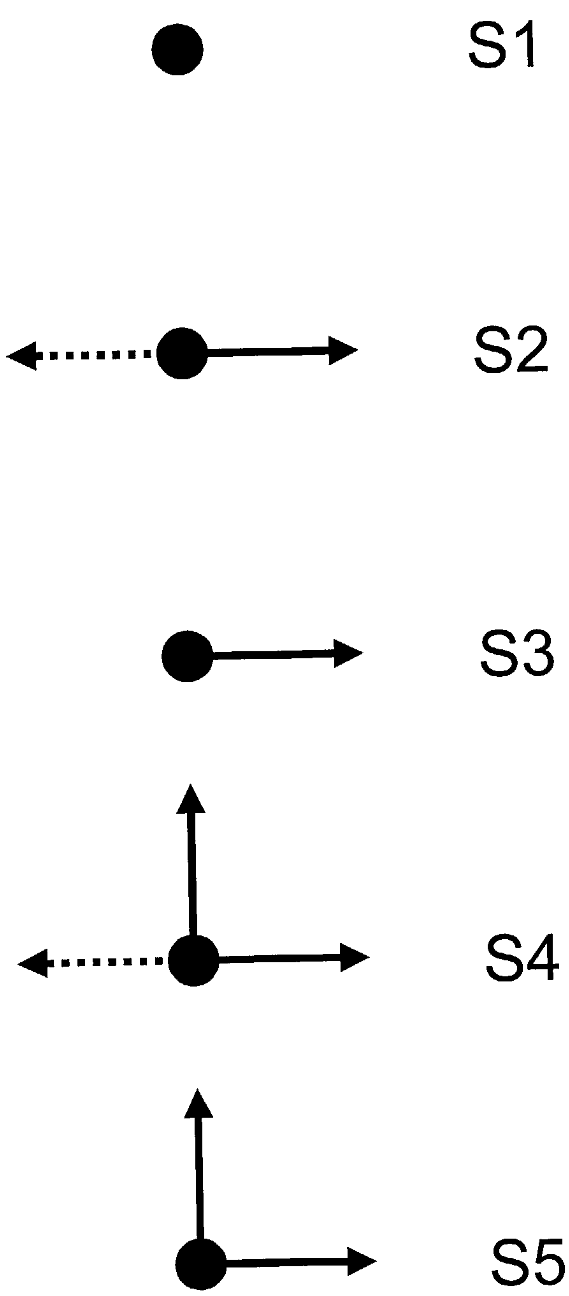

Figure 4

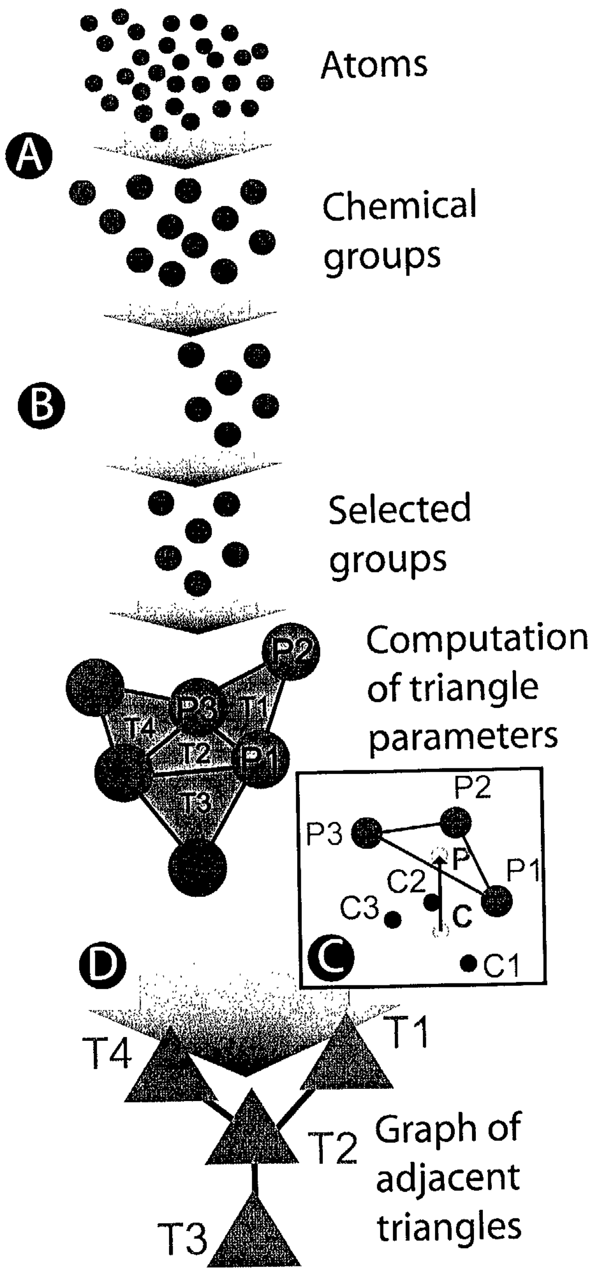
Atoms
A
Chemical groups
B
Selected groups
Computation of triangle parameters
P2
P3
T1
T4
T2
P1
T3
P2
P3
P
P1
C2
C3
C
C
C1
D
T4
T1
T2
Graph of adjacent triangles
T3

Figure 5

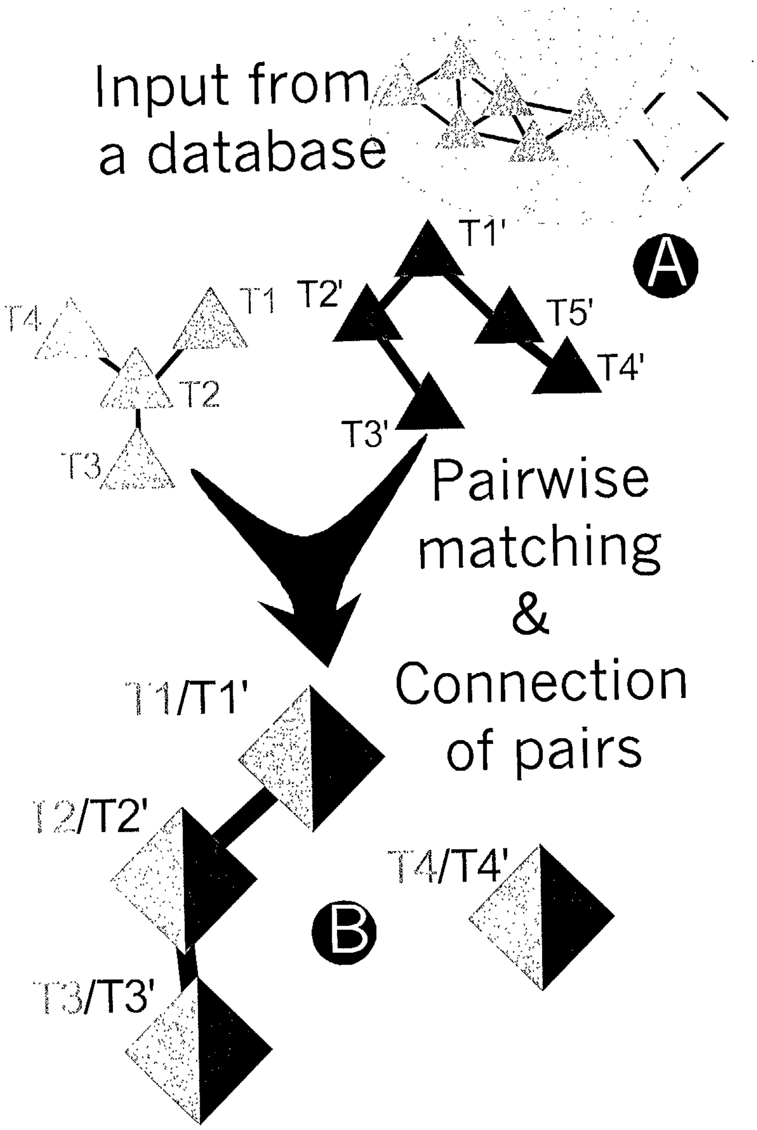
Input from a database
T1'
A
T2'
T5'
T4
T1
T4'
T2
T3'
T3
Pairwise matching & Connection of pairs
T1/T1'
T2/T2'
T4/T4'
B
T3/T3'

Figure 6

Method refinement

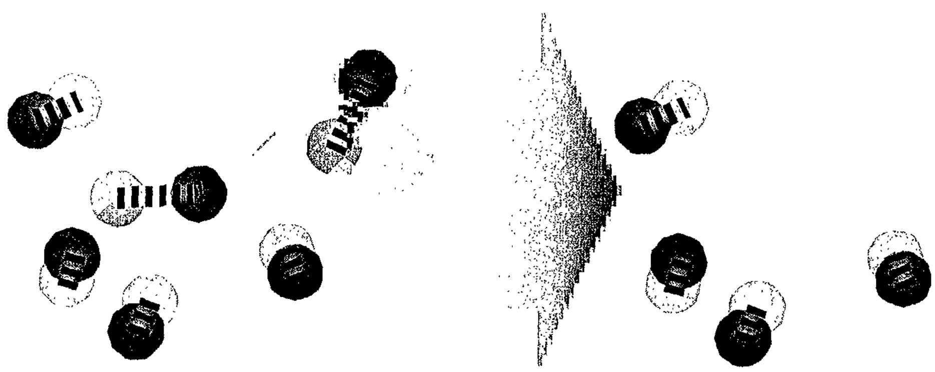

Figure 7

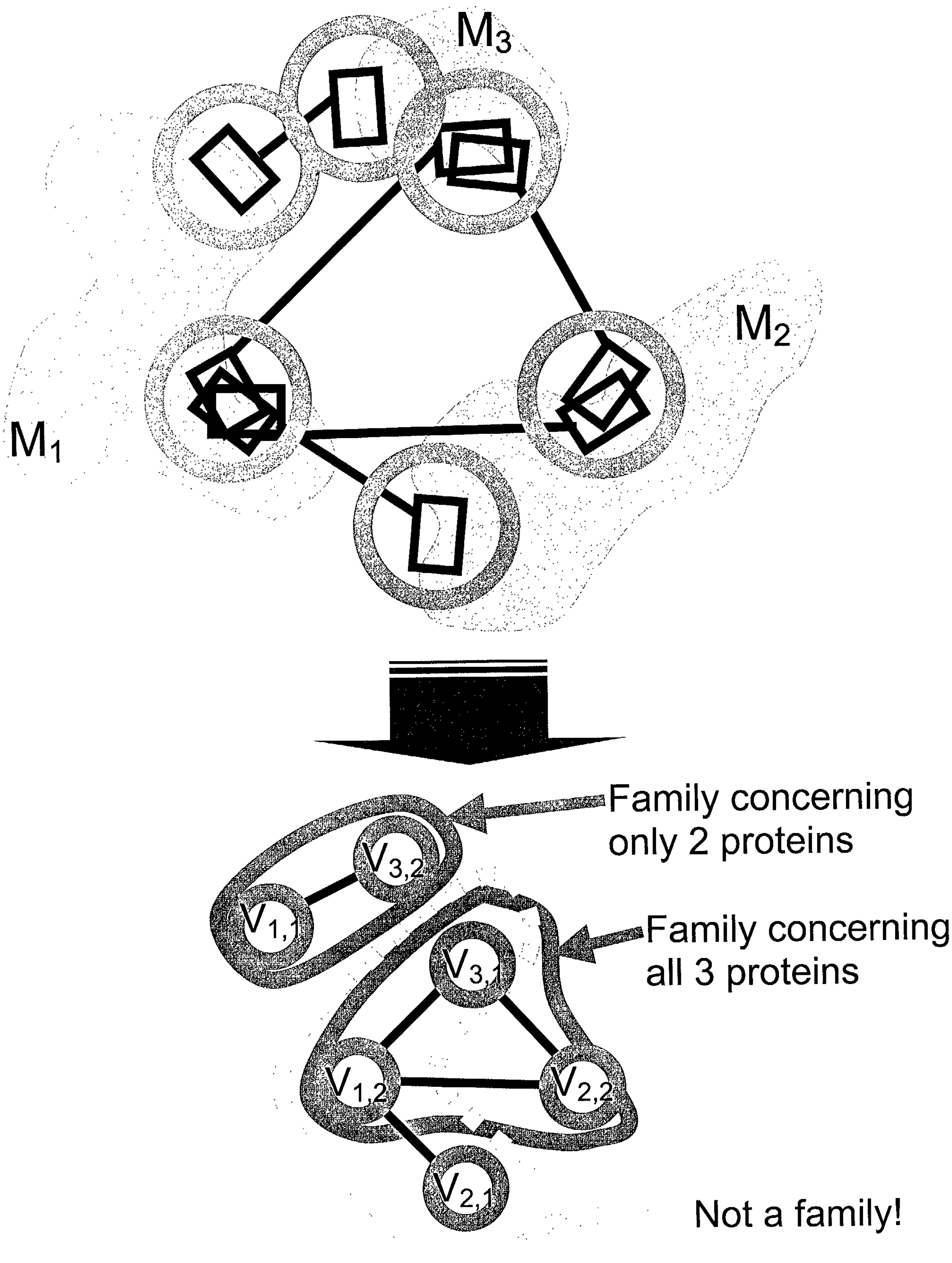
M3
M2
M1
Family concerning only 2 proteins
V3,2
V1,1
Family concerning all 3 proteins
V3,1
V1,2
V2,2
V2,1
Not a family!

Figure 8

Proteases sequences

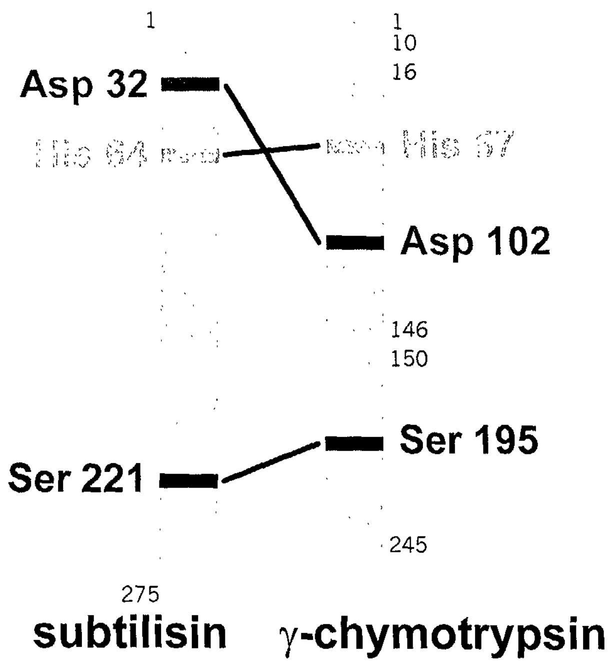

Figure 9

Proteases file

```
[
Comparison result of (1SBC,1AFQ):
---------------------------- Patch number 1 ---
Number of groups: 5
Score = 1.249 [RMSD = 0.712] [penalty = 0.537]

Selected pairs of groups:
        acyl   ASP   32 | 0.66 |
        acyl B ASP  102 | 0.67 | 0.010 |  0.871

    aromatic   HIS   64 | 0.56 |
    aromatic B HIS   57 | 0.56 | 0.003 |  0.562

    ammonium   HIS   64 | 0.54 |
    ammonium B HIS   57 | 0.55 | 0.006 |  0.432

     glycine   GLY  127 | 0.50 |
     glycine C GLY  216 | 0.55 | 0.056 |  0.176

    hydroxyl   SER  221 | 0.57 |
    hydroxyl C SER  195 | 0.59 | 0.015 |  1.116
]
```

Figure 10

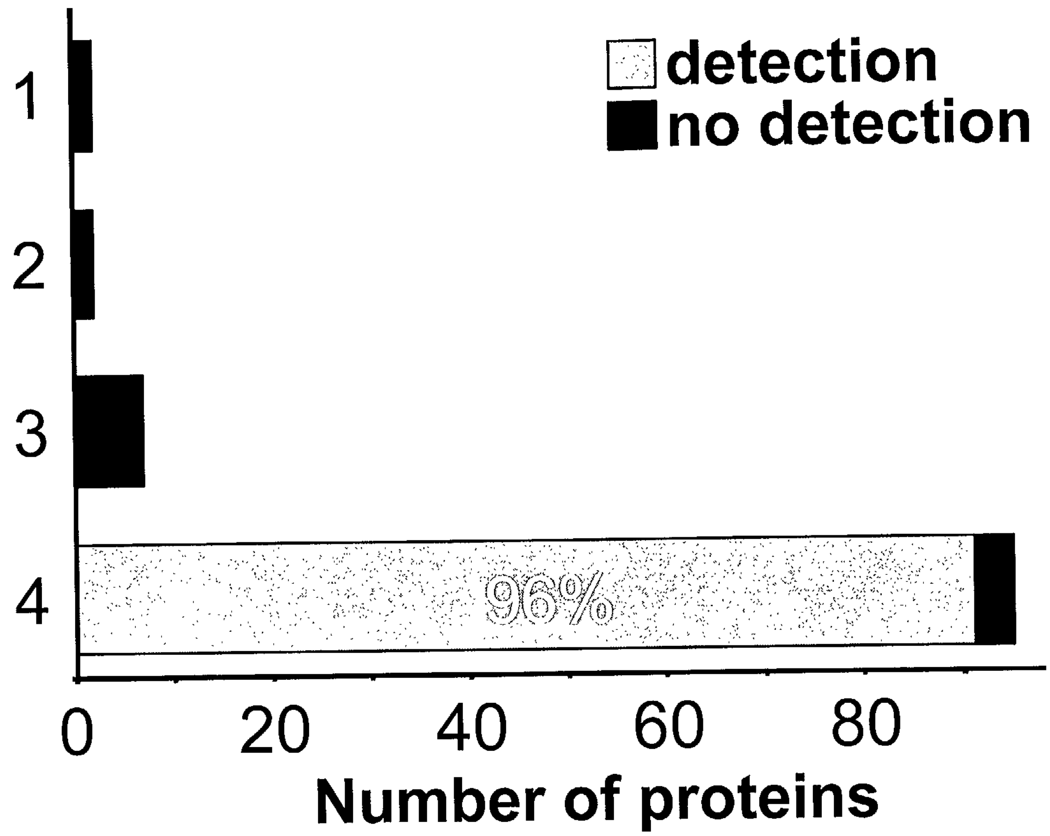
detection
no detection
1
2
3
4
96%
0
20
40
60
80
Number of proteins

Figure 11

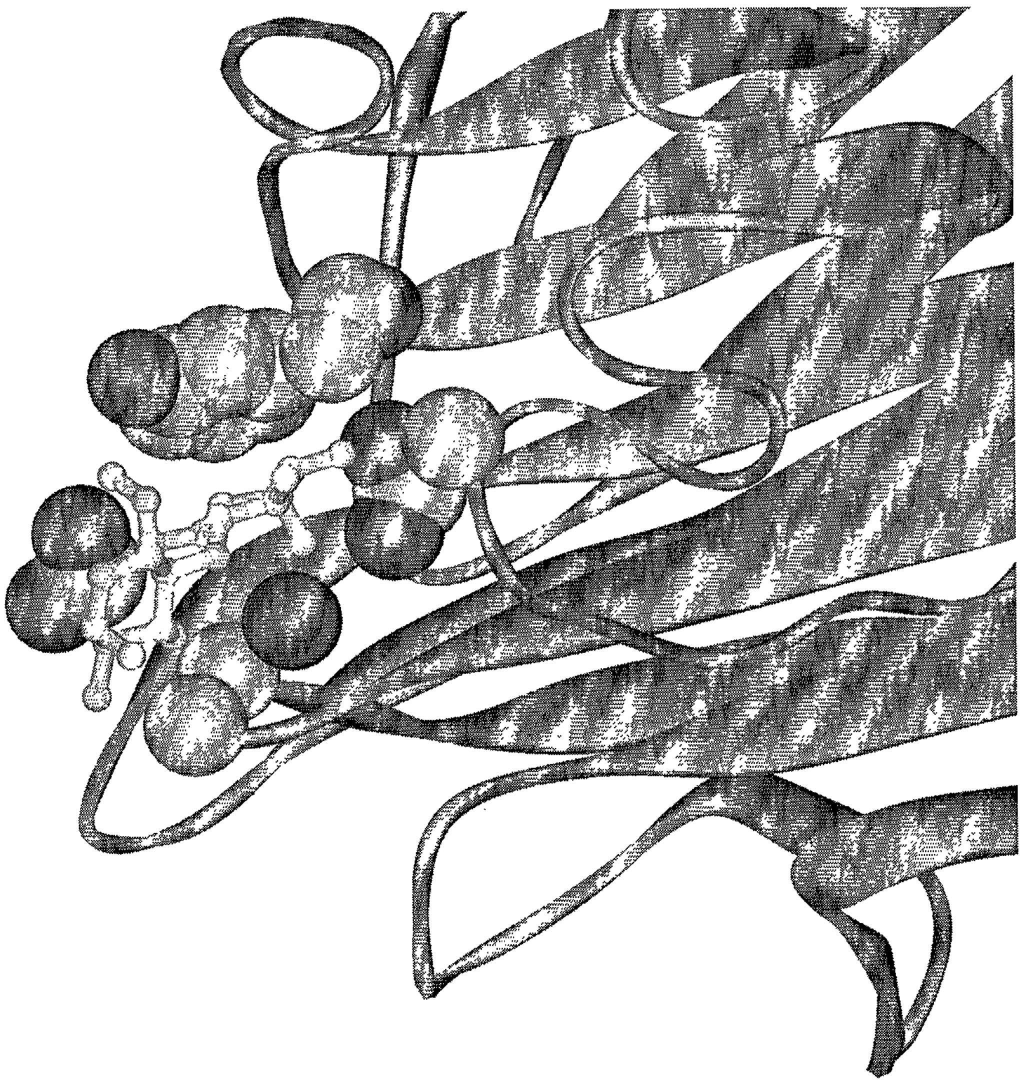

Figure 12

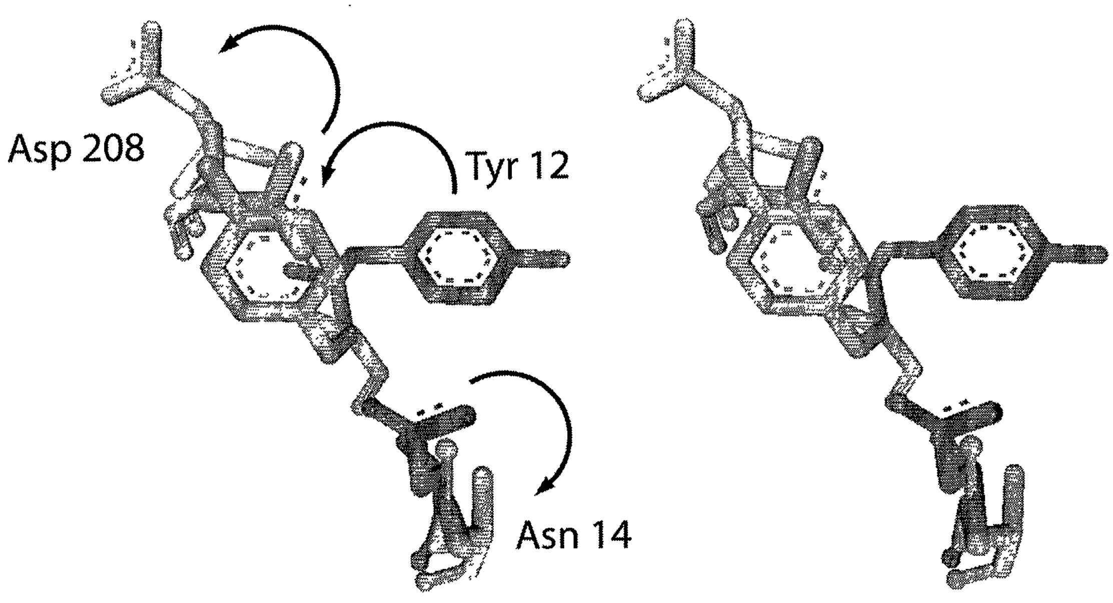

European Patent Office

# EUROPEAN SEARCH REPORT

Application Number
EP 02 29 1407

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 2001/034580 A1 (FETROW JACQUELYN S ET AL) 25 October 2001 (2001-10-25)<br>* abstract *<br>* paragraph '0054! - paragraph '0057! *<br>--- | 1-26 | G06F19/00 |
| A | WILLET P: "CHEMICAL SIMILARITY SEARCHING" JOURNAL OF CHEMICAL INFORMATION AND COMPUTER SCIENCES, AMERICAN CHEMICAL SOCIETY, COLOMBUS,OHIO, US,<br>vol. 38, no. 6, November 1998 (1998-11), pages 983-996, XP000890390<br>ISSN: 0095-2338<br>* the whole document *<br>--- | 1-26 | |
| A | COMBET C ET AL: "Geno3D: automatic comparative molecular modelling of protein"<br>BIOINFORMATICS, 'Online!<br>vol. 18, no. 1,<br>1 January 2002 (2002-01-01), pages 213-214, XP002243511<br>Retrieved from the Internet:<br><URL:http://bioinformatics.oupjournals.org/cgi/reprint/18/1/213.pdf><br>'retrieved on 2003-06-02!<br>* the whole document *<br>--- | 1-26 | TECHNICAL FIELDS SEARCHED (Int.Cl.7)<br>G06F |
| A | EP 0 849 690 A (BIOMOLECULAR ENGINEERING RESEA) 24 June 1998 (1998-06-24)<br>* abstract; claim 1 *<br>--- | 1-26 | |
| A | PATENT ABSTRACTS OF JAPAN<br>vol. 1996, no. 02,<br>29 February 1996 (1996-02-29)<br>& JP 07 287717 A (FUJITSU LTD),<br>31 October 1995 (1995-10-31)<br>* abstract *<br>---<br>-/-- | 1-26 | |

The present search report has been drawn up for all claims

| Place of search | Date of completion of the search | Examiner |
|---|---|---|
| THE HAGUE | 5 June 2003 | Filloy García, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 02 29 1407

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 93 01484 A (UNIV CALIFORNIA)<br>21 January 1993 (1993-01-21)<br>* abstract *<br>----- | 1-26 | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 5 June 2003 | Filloy García, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 29 1407

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2003

| Patent document cited in search report | Publication date | Patent family member(s) | Publication date |
|---|---|---|---|
| US 2001034580 A1 | 25-10-2001 | AU 4218599 A | 13-12-1999 |
| | | AU 4218799 A | 14-03-2000 |
| | | CA 2340284 A1 | 02-03-2000 |
| | | CN 1328601 T | 26-12-2001 |
| | | EP 1108055 A1 | 20-06-2001 |
| | | JP 2002523057 T | 30-07-2002 |
| | | WO 9961654 A1 | 02-12-1999 |
| | | WO 0011206 A1 | 02-03-2000 |
| EP 0849690 A | 24-06-1998 | JP 10185925 A | 14-07-1998 |
| | | EP 0849690 A2 | 24-06-1998 |
| | | US 6125331 A | 26-09-2000 |
| JP 07287717 A | 31-10-1995 | JP 3235763 B2 | 04-12-2001 |
| | | US 6453064 B1 | 17-09-2002 |
| WO 9301484 A | 21-01-1993 | AU 2408292 A | 11-02-1993 |
| | | WO 9301484 A2 | 21-01-1993 |
| | | US 5436850 A | 25-07-1995 |

EPO FORM P0459

For more details about this annex : see Official Journal of the European Patent Office, No. 12/82